(19) Europäisches Patentamt
European Patent Office
Office européen des brevets

(11) Veröffentlichungsnummer: **0 249 013 B1**

# EUROPÄISCHE PATENTSCHRIFT

(12)

(45) Veröffentlichungstag der Patentschrift:
06.02.91 Patentblatt 91/06

(51) Int. Cl.⁵: **C07H 15/04**

(21) Anmeldenummer: 87105741.0

(22) Anmeldetag: 16.04.87

(54) Verfahren zur Herstellung von Alkyloligoglycosiden.

(30) Priorität: 12.06.86 DE 3619796

(43) Veröffentlichungstag der Anmeldung:
16.12.87 Patentblatt 87/51

(45) Bekanntmachung des Hinweises auf die
Patenterteilung:
06.02.91 Patentblatt 91/06

(84) Benannte Vertragsstaaten:
AT BE DE ES FR GB IT NL

(56) Entgegenhaltungen:
EP-A- 0 092 875
DE-A- 1 905 523
FR-A- 2 017 240
GB-A- 2 132 202
US-A- 3 219 656
THE JOURNAL OF THE AMERICAN OIL CHE-
MISTS' SOCIETY, Band 47, Nr. 5, Mai 1970,
Seiten 162-167, The American Oil Chemists'
Society, Chicago, Illinois, US; F.A. HUGHES et
al.: "Physical and functional properties of
some higher alkyl polyglycosides"

(73) Patentinhaber: HÜLS
AKTIENGESELLSCHAFT
Patentabteilung / PB 15 - Postfach 13 20
D-4370 Marl 1 (DE)

(72) Erfinder: Lüders, Harald, Dr.
Langeoogstrasse 81
D-4350 Recklinghausen (DE)

## Beschreibung

Alkyloligoglycoside können ganz oder teilweise auf Basis von nachwachsenden Rohstoffen hergestellt werden. Wegen ihrer geringen Toxizität, ihrer sehr guten biologischen Abbaubarkeit und ihrer interessanten anwendungstechnischen Eigenschaften beanspruchen diese Verbindungen ein großes Interesse. Das Eigenschaftsbild der Alkyloligoglycoside wird nach F. A. Hughes und B. W. Lew, J.A.O.C.S. 47 (1970), 162 stark von ihrem Oligomerisationsgrad bestimmt. Aus diesem Grunde ist man an Verfahren interessiert, durch die Alkyloligoglycoside mit bestimmten, mittleren Oligomerisationsgraden gezielt hergestellt werden können.

Nach der Fischer-Glycosidsynthese kann Glucose mit hydrophilen Alkoholen wie Methanol oder Ethanol unter Säurekatalyse glatt zu einem Alkylglucosid umgesetzt werden. Bei hydrophoben Alkoholen treten dagegen Löslichkeitsprobleme auf.

Nach US-PS 3 219 656 werden höhere Alkylglucoside dadurch erhalten, daß man zunächst Butylglucosid herstellt, das dann mit einem höheren Alkohol umglucosidiert wird. Dieses Verfahren erfordert große Mengen an Kationentauscher.

Nach DE-OS 19 43 689 werden höhere Alkyloligosaccharide aus Butylglucosid durch Umacetalisierung mit Alkoholen mit 11 bis 32 C-Atomen unter Säurekatalyse hergestellt. Dabei ist der Oligomerisationsgrad umgekehrt proportional zu den pro Mol Butylglucosid verwendeten Molen Alkohol.

Diese reziproke Abhängigkeit wird auch in DE-OS 19 05 523 bestätigt. Bei der Umacetalisierung werden hier Alkohol-Monosaccharid-Molverhältnisse von 0,05 bis 12 eingestellt, wodurch Alkyloligoglucoside mit durchschnittlich 25 bis 1,4 Glucoseeinheiten pro Molekül erhalten werden.

Demnach müssen für hohe Oligomerisationsgrade so niedrige Alkohol-Kohlenhydrat-Verhältnisse gewählt werden, daß der Reaktionsverlauf wegen Pastenbildung beeintrachtigt wird. Auf der anderen Seite wird bei niedrigen Oligomerisationsgraden mit sehr hohen Alkoholüberschüssen gearbeitet, die nach beendeter Reaktion abdestilliert werden müssen. Wegen der sehr unterschiedlichen anzuwendenden Alkoholmengen ist deshalb eine kontinuierliche Produktionsweise einer Produktpalette mit Oligomerisationsgraden beispielsweise von 1,5 bis 5 in derselben Anlag nur mit großem verfahrenstechnischen Aufwand realisierbar.

In EP-A-0 092 875 wird eine säurekatalysierte Umglycosidierung von kurzkettigen Alkylmonosacchariden mit Fettalkoholen mit 12 bis 18 Kohlenstoffatomen zu langkettigen Alkylpolysacchariden durchgeführt. Dabei wird der Katalysator zerstört, wenn mindestens 90% der kurzkettigen Alkylmonosaccharide umgesetzt sind. Der Polymerisationsgrad ergibt sich danach hauptsächlich aus dem Saccharidmonomer-Fettalkohol-Verhältnis.

Die als Katalysator zugegebene Säure beeinflußt nach US-PS 3 839 318 und US-PS 4 465 828 lediglich die Reaktionsgeschwindigkeit. Ein Einfluß der Katalysatorkonzentration auf den Oligomerisationsgrad war nicht bekannt. Er war auch nicht zu erwarten. Denn in US-PS 4 223 129 wurde festgestellt, daß die Katalysatorkonzentration bei der Herstellung von Methyloligoglycosiden keinen Einfluß auf den Oligomerisationsgrad besitzt.

Aufgabe der vorliegenden Erfindung ist es, ein vereinfachtes Verfahren zur Herstellung von Alkyloligoglycosiden ait Alkylgruppen mit bis zu 24 C-Atomen bereitzustellen. Bestimmte Oligomerisationsgrade sollen dabei leicht und reproduzierbar eingestellt werden können. Die Reaktionsgemische sollen für technisch interessante Oligomerisationsgrade stets gut rührbar sein. Außerdem sollen keine hohen Alkoholüberschüsse zum Einsatz kommen.

Oberraschenderweise wird die Aufgabe dadurch gelöst, daß man die Umglycosidierung zu Alkyloligoglycosiden in einer gut handhabbaren Reaktionsmischung durchführt und dabei den Oligomerisationsgrad durch Zugabe einer Säuremenge von 2 bis 200 Milliäquivalenten Säure pro kg Alkohol einstellt.

Als Ausgangsstoffe für die Umglycosidierung werden Alkylglycoside, Alkyloligoglycoside oder deren Mischungen eingesetzt. Sie können Monosaccharide wie Glucose, Mannose, Galactose, Fructose, Ribose oder Xylose enthalten. Oer Alkylanteil der Ausgangsstoffe enthält mindestens 3 C-Atome weniger als der eingesetzte Alkohol. Der Alkylanteil weist vorzugsweise 2 bis 6 C-Atome auf. Vorzugsweise werden Butylglycoside, Butyloligoglycoside oder deren Gemische eingesetzt. Besonders bevorzugte Ausgangsverbindungen sind die Glucosederivate n-Butylglucosid, n-Butyloligoglucoside und deren Mischungen.

Diese Produkte erhält man beispielsweise bei der säurekatalysierten Alkoholyse von Stärke oder Stärkehydrolyseprodukten. Die Alkylglycoside und Alkyloligoglycoside können in reiner Form oder im Gemisch mit dem Alkohol, der zu ihrer Herstellung verwendet wurde, eingesetzt werden.

Zur Umglycosidierung werden als zweite Komponente primäre Alkohole mit 4 bis 24 C-Atomen verwendet. Vorzugsweise enthalten die Alkohole 8 bis 20 C-Atome. Beispiele dafür sind native Tensidalkohole oder Fettalkohole, wie sie unter anderem auch bei der Hydrierung von Fettsäuren und Fettsäurederivaten entstehen, aber auch vollsynthetische Ziegleralkohole und Oxoalkohole. Es können auch Alkoholgemische eingesetzt werden. Die Alkohole können Verzweigungen in der Kohlenstoffkette aufweisen. Vorzugsweise werden jedoch lineare Alkohole verwendet.

Die Umglycosidierung wird durch Säuren katalysiert. Gleichzeitig dient der Säurezusatz zur Regulierung des Oligomerisationsgrades. Man verwendet dabei starke Mineralsäuren oder organische Sulfonsäuren, wobei Schwefelsäure bevorzugt wird.

Säure kann bereits im Ausgangsmaterial vorhanden sein. Die wirksame Säurekonzentration wird daher entweder durch entsprechendes Zudosieren von Säure oder durch partielle Neutralisation von vorhandener Säure eingestellt.

Zur Umglycosidierung werden Alkohol und Alkylglycosid bzw. Alkyloligoglycosid in Molverhältnissen von 10 : 1 bis 0,5 : 1 (berechnet als Mol Alkohol pro Mol Saccharideinheit) eingesetzt. Vorzugsweise liegen die Molverhältnisse bei 5 : 1 bis 1 : 1.

Die bei der Umglycosidierung freigesetzten und ggf. neben dem Ausgangsmaterial vorhandenen kurzkettigeren Alkohole werden destillativ aus dem Reaktionsgemisch entfernt. Günstig ist es, wenn der Siedepunkt des kurzkettigeren Alkohols mehr als 30°C unter dem Siedepunkt des zu Umglycosidierung zugesetzten Alkohols liegt.

Der kurzkettigere Alkohol wird bei einer Reaktionstemperatur von 80 bis 140°C abdestilliert, wobei auch Vakuum angelegt werden kann. Dabei kann man auch ein Inertgas durch die Schmelze perlen lassen.

Nach beendeter Umglycosidierung wird die Säure durch eine Lauge neutralisiert. Überschüssiger Alkohol wird dann im Vakuum destilliert, wobei man wegen der Gefahr einer Zersetzung des Produktes 150°C nicht überschreitet.

Das Verfahren weist folgende Vorteile auf :

1. Die Herstellung von Alkyloligoglycosiden mit verschiedenen mittleren Oligomerisationsgraden kann bei gleichbleibendem Eduktverhältnis Alkohol/Kohlenhydrat durchgeführt werden. So kann man beispielsweise bei einem Molverhältnis Laurylalkohol/n-Butylglucosid von 1,7 : 1 die Schwefelsäuremenge von 13 auf 80 Milliäquivalente pro kg Laurylalkohol steigern. Dadurch erhöht sich der mittlere Oligomerisationsgrad im gebildeten Lauryloligoglucosid von 2,2 auf 5.

2. Bei kontinuierlicher Fahrweise bleiben die Stoffströme bei Äderung des mittleren Oligomerisationsgrades praktisch gleich.

3. Bei der Herstellung von Alkyloligoglycosiden mit hohen mittleren Oligomerisationsgraden bleiben die Reaktionsgemische leicht rührbar.

4. Bei der Herstellung von Alkyloligoglycosiden mit niedrigen mittleren Oligomerisationsgraden kommt man mit relativ wenig Alkohol aus.

5. Die Säurekonzentration kann sehr einfach eingestellt werden. Durch sie kann der mittlere Oligomerisationsgrad im praktisch interessierenden Bereich bis 15 wirkungsvoll gesteuert werden.

Die nach dem erfindungsgemäßen Verfahren hergestellten Alkyloligoglycoside sind als Tenside, Schmiermittel, Farbhilfsstoffe, als ungiftige Lebensmittelemulgatoren, Anfeuchter oder als Polyole zur Herstellung von Polyurethanen geeignet.

## Beispiele

Der mittlere Oligomerisationsgrad N, die durchschnittliche Anzahl von Monosaccharideinheiten pro Molekül, wird nach folgendem Verfahren bestimmt :

10 g gereinigtes Alkyloligoglycosid werden mit 100 g Ethylenglykol und 5 ml 20%iger Schwefelsäure unter Rühren 5 Stunden auf 110°C erhitzt.Das Gemisch wird dann mit 100 ml Wasser verdünnt, worauf der durch Glycolyse entstandene Alkohol zweimal mit 50 ml Hexan extrahiert wird. Aus den vereinigten Extrakten wird die erhaltene Alkoholmenge A gaschromatographisch bestimmt. Die Kohlenhydratmenge K (Anhydrosaccharidmenge) ist dann :

$$K = 10 - A \ (g)$$

Aus der nun bekannten mengenmäßigen Zusammensetzung des Alkyloligoglycosids und den Molmassen der beteiligten Komponenten kann der mittlere Oligomerisationsgrad N nach folgender Formel mit hinreichender Genauigkeit errechnet werden :

$$N = \frac{K/M_K}{A/M_A}$$

Dabei ist $M_K$ die (mittlere) Molmasse der zugrundeliegenden Saccharideinheiten und $M_A$ die (mittlere) Molmasse des verwendeten Alkohols.

Beispiel 1

100 g n-Butylglucosid werden bei 100°C unter Rühren in 250 g Laurylalkohol gelöst und mit 1,6 ml 2 N butanolischer Schwefelsäure versetzt. Der Ansatz wird in einer Destillationsapparatur erhitzt und 1 Stunde bei 10 mbar und 130°C gehalten, wobei n-Butanol abdestilliert. Der Ansatz wird auf 100°C abgekühlt, bei Normaldruck mit 1,6 ml 2 N Natronlauge neutralisiert, mit 0,1 g Natriumhydrogencarbonat gepuffert und dann 15 Minuten nachgerührt. Leichtflüchtige Bestandteile werden im Wasserstrahlvakuum abdestilliert. Der Ansatz wird dann filtriert, worauf der überschüssige Laurylalkohol im Ölvakuum abdestilliert wird.
Es wird ein pastöses Laryloligoglucosid mit einem mittleren Oligomerisationsgrad N=2,2 Glucoseeinheiten erhalten.

Beispiel 2 bis 9

Es wird wie in Beispiel 1 verfahren. Die jeweils zu 100 g n-Butylglycosid zugegebenen Einsatzmengen und die mittleren Oligomerisationsgrade N der Produkte sind in Tabelle 1 zusammengestellt.

· Tabelle 1

| Beispiel | Alkohol g | 2N $H_2SO_4$ ml | 2N NaOH ml | Oligomerisations- grad N |
|---|---|---|---|---|
| 1 | 250 g Laurylalkohol | 1,6 | 1,6 | 2,2 |
| 2 | 250 g " | 2,5 | 2,5 | 2,7 |
| 3 | 250 g " | 5,0 | 5,0 | 4,2 |
| 4 | 250 g " | 10,0 | 10,0 | 5,0 |
| 5 | 250 g Ziegleralkohol[1] | 1,6 | 1,6 | 3,9 |
| 6 | 250 g " | 10,0 | 10,0 | 7,0 |
| 7 | 500 g Laurylalkohol | 10,0 | 10,0 | 1,8 |
| 8 | 500 g " | 20,0 | 20,0 | 3,2 |
| 9 | 500 g " | 40,0 | 40,0 | 3,7 |

1) Gemisch aus etwa 1% Decanol, 72% Dodecanol, 25% Tetradecanol und 1% Hexadecanol, ALFOL[R] 1214 von Condea, D-2212 Brunsbüttel

**Ansprüche**

1. Verfahren zur Herstellung von Alkyloligoglycosiden, deren Alkylgruppen bis zu 24 C-Atome aufweisen, aus Alkylglycosiden oder Alkyloligoglycosiden und Alkoholen, dadurch gekennzeichnet, daß man den mittleren Oligomerisationsgrad mit Hilfe einer Säuremenge von 2 bis 200 Milliäquivalenten Säure pro kg Alkohol einstellt und daß die Alkohole mindestens 3 C-Atome mehr aufweisen als die Alkylreste der eingesetzten Alkylglycoside und Alkyloligoglycoside.

2. Verfahren nach Anspruch 1, dadurch gekennzeichnet, daß der mittlere Oligomerisationsgrad mit Schwefelsäure eingestellt wird.

3. Verfahren nach den Ansprüchen 1 und 2, dadurch gekennzeichnet, daß die Alkohole 8 bis 20 C-Atome und die Alkylreste der eingesetzten Alkylglycoside und Alkyloligoglycoside 2 bis 6 C-Atome aufweisen.

4. Verfahren nach den Ansprüchen 1 bis 3, dadurch gekennzeichnet, daß man von Butylglycosiden, Butyloligoglycosiden oder deren Gemischen ausgeht.

5. Verfahren nach Anspruch 4, dadurch gekennzeichnet, daß man von n-Butylglucosid, n-Butyloligoglucosiden oder deren Gemischen ausgeht.

6. Verfahren nach den Ansprüchen 1 bis 5, dadurch gekennzeichnet, daß 0,5 bis 10 Mol Alkohol pro Mol Saccharideinheit eingesetzt werden.

7. Verfahren nach Anspruch 6, dadurch gekennzeichnet, daß 1 bis 5 Mol Alkohol pro Mol Saccharideinheit eingesetzt werden.


## Claims

1. A process for the preparation of alkyl oligoglycosides, the alkyl groups of which have up to 24 C atoms, from alkyl glycosides or alkyl oligoglycosides and alcohols, characterized in that the average degree of oligomerization is adjusted with the aid of an amount of acid of from 2 to 200 milliequivalents of acid per kg of alcohol and that the alcohols have at least 3 C atoms more than the alkyl radicals of the alkyl glycosides and alkyl oligoglycosides employed.

2. A process according to claim 1, characterized in that the average degree of oligomerization is adjusted with the aid of sulphuric acid.

3. A process according to either of claims 1 and 2, characterized in that the alcohols have 8 to 20 C atoms and the alkyl radicals of the alkyl glycosides and alkyl oligoglycosides employed have 2 to 6 C atoms.

4. A process according to any of claims 1 to 3, characterized in that the process commences from butyl glycosides, butyl oligoglycosides or mixtures thereof.

5. A process according to claim 4, characterized in that the process commences from n-butyl glucoside, n-butyl oligoglucosides or mixtures thereof.

6. A process according to any of claims 1 to 5, characterized in that 0.5 to 10 mol of alcohol are employed per mol of saccharide unit.

7. A process according to claim 6, characterized in that 1 to 5 mol of alcohol are employed per mol of saccharide unit.


## Revendications

1. Procédé de préparation d'alkyl-oligo-glycosides, dont les groupes alkyle présentent jusqu'à 24 atomes de carbone, à partir d'alkyl-glycosides ou d'alkyl-oligoglycosides et d'alcools, caractérisé par le fait que l'on établit le degré moyen d'oligomérisation à l'aide d'une quantité d'acide de 2 à 200 milli-équivalents d'acide par kilogramme d'alcool at que les alcools présentent au moins 3 atomes de carbone de plus que les radicaux alkyle des alkyl-glycosides et des alkyl-oligo-glycosides qui sont utilisés.

2. Procédé selon la revendication 1, caractérisé par le fait que le degré moyen d'oligomérisation est établi avec de l'acide sulfurique.

3. Procédé selon les revendications 1 et 2, caractérisé que les alcools présentent de 8 à 20 atomes de carbone et que les restes alkyle des alkyl-glycosides et des alkyl-oligo-glycosides présentent de 2 à 6 atomes de carbone.

4. Procédé selon les revendications 1 à 3, caractérisé par le fait que l'on part de butyl-glycosides, de butyl-oligo-glycosides ou de leurs mélanges.

5. Procédé selon la revendication 4, caractérisé par le fait que l'on part de n-butyl-glycoside, de n-butyl-oligo-glycosides ou de leurs mélanges.

6. Procédé selon les revendications 1 à 5, caractérisé par le fait que l'on utilise de 0,5 à 10 moles d'alcool par mole d'unité saccharide.

7. Procédé selon la revendication 6, caractérisé par le fait que l'on utilise de 1 à 5 moles d'alcool par mole d'unité saccharide.